# EUROPÄISCHE PATENTSCHRIFT

(11) **EP 3 721 975 B1**
(45) Veröffentlichungstag und Bekanntmachung des Hinweises auf die Patenterteilung: **21.09.2022**
(21) Anmeldenummer: 20160279.4
(22) Anmeldetag: 28.02.2020
(51) Int. Cl.: B01D 65/02, A61L 2/20, B01D 69/06, F26B 5/06

(54) **VERFAHREN ZUR HERSTELLUNG EINES PARTIKELFREIEN, STERIL-VERPACKTEN FILTERPRODUKTS**
METHOD FOR PRODUCING A PARTICLE-FREE STERILE PACKAGED FILTER PRODUCT
PROCÉDÉ DE FABRICATION D'UN PRODUIT FILTRANT SANS PARTICULES, CONDITIONNÉ STÉRILISÉ

(30) Priorität: 11.04.2019 DE 102019109635
(43) Veröffentlichungstag der Anmeldung: 14.10.2020
(73) Patentinhaber: Kögel, Jürgen, 67365 Schwegenheim (DE)
(72) Erfinder: Kögel, Jürgen, 67365 Schwegenheim (DE)
(74) Vertreter: Christ, Niko

(56) Entgegenhaltungen:
- EP-A1- 2 157 387
- WO-A2-2017/137637
- CN-A- 106 643 116
- DE-U1- 29 908 080
- US-B1- 6 517 526
- Sartorius: "Products for Microbiological Control 2 Microbiological Analysis Content", Sartorius, 1. Januar 2016 (2016-01-01), XP055639301, Gefunden im Internet: URL:http://citenpl.internal.epo.org/wf/web /citenpl/citenpl.html [gefunden am 2019-11-05]

## Beschreibung

Die vorliegende Erfindung betrifft ein Verfahren zur Herstellung eines partikelfreien, steril verpackten Filterprodukts, umfassend ein Filtermaterial, welches zu einem Filtertuch zugeschnitten ist.

Ein steriles, wenngleich nicht partikelfreies, Filterprodukt ist bereits aus der CN 106 643 116 A vorbekannt. Ebenfalls sind solche Filterprodukte aus der Veröffentlichung Sartorius: "Products for Microbiological Control 2 Microbiological Analysis Content", Sartorius, 1. Januar 2016, XP055639301.

Weiterer relevanter Stand der Technik ergibt sich aus der DE 299 08 080 U1 und der WO 2017/137637 A2.

Derartige Filterprodukte sind bereits im Stand der Technik bekannt. So kennt man beispielsweise Wannensysteme zur Verwendung in Gefriertrocknungsverfahren, welche im Rahmen der Gefriertrocknung, der so genannten Lyophilisation, eingesetzt werden. Ein solches Wannensystem umfasst eine Wanne zur Aufgabe des Lyophilisats, über welchem in einem Halter ein Filtertuch angeordnet wird. Hierbei ist darauf zu achten, dass ein solches Filtertuch vorher sterilisiert wird, um einen Einschluss von Keimen zu vermeiden, welche das Lyophilisat verunreinigen würde. In einer solchen Lösung ist das Wannensystem beispielsweise aus Edelstahl gefertigt und damit wiederverwendbar, während allerdings das Filtertuch nach der Benutzung ausgetauscht und entsorgt werden muss.

Allerdings erfordert dies entsprechende Kapazitäten zur Sterilisierung am Verwendungsort, die nicht zwangsläufig gegeben sind. Daher wird im Alltag entweder mit einem erheblichen Aufwand, oder aber mit einer verminderten Qualität gearbeitet, wo möglicherweise die Sterilisiation des Filtertuchs außer Acht gelassen wird.

Ferner ist unter dem Namen 'Lyoguard' ein Produkt bekannt, bei dem ein dort verwendetes Kunststoff-Wannensystem mit einem Filtertuch direkt verschweißt wird. Eine solche Lösung ist jedoch nicht wiederverwendbar und verursacht daher große Mengen an sich vermeidbaren Abfalls, da hier das System aus Filtertuch und Wanne eine Einheit bildet und vollständig nicht mehrfach verwendbar ist. Dieses bekannte System stellt ein Single-use-System dar.

Vor diesem Hintergrund liegt der vorliegenden Erfindung die Aufgabe zu Grunde, ein Verfahren zur Herstellung eines Filterprodukts mit einem möglichst geringen Aufwand aber gleichzeitig in hoher Qualität vorzuschlagen.

Gelöst wird diese Aufgabe mit einem Verfahren zur Herstellung eines partikelfreien, steril verpackten Filterprodukts gemäß den Merkmalen des unabhängigen Anspruchs 1. Weitere, sinnvolle Weiterentwicklungen eines solchen Herstellungsverfahrens sowie eines solchermaßen hergestellten Filterprodukts können den jeweils nachfolgenden, abhängigen Ansprüchen entnommen werden.

Erfindungsgemäß ist es vorgesehen, ein Filterprodukt in möglichst hoher Qualität vorzubereiten und in einer sterilen Verpackung vorzuhalten, aus der es bei Bedarf entnommen und seiner Verwendung zugeführt werden kann. Hierbei ist das Filtermaterial als solches bereits bekannt, so dass es einzig auf den Herstellungsvorgang des Filterprodukts als solches ankommt. Wie im Stand der Technik bereits realisiert, wird ein Filtermaterial ausgewählt, welches in einem ersten Schritt zu einem Filtertuch, vorzugsweise in einem Reinraum der Reinraumklasse ISO 6, zugeschnitten wird. Dieses Filtertuch wird sodann unter Reinraumbedingungen, vorzugsweise derselben Reinraumklasse ISO 6, weiterverarbeitet. Hierbei wird es zunächst in einem Ultraschallbad mechanisch gereinigt. Durch die Ultraschallanregung entstehen in dem Reinigungsmedium Kavitationsblasen, welche sich mit Dampf füllen und unter Einwirkung äußeren Drucks im Rahmen einer Blasenimplosion zusammenfallen. Soweit dieses Platzen der Dampfblasen im Bereich von Verunreinigungen am Filtertuch geschieht, sprengt diese die Verunreinigung von dem Filtertuch ab und sorgt dafür, dass die Verunreinigung in dem Reinigungsmedium gelöst oder zumindest fortgetragen wird. Das Reinigungsmedium als solches wird im Zuge der Reinigung fortwährend gefiltert.

Mehrere Filtertücher können besonders vorteilhaft in einem Korb schichtweise beabstandet nebeneinander angeordnet sein, so dass in einem Bad mehrere Filtertücher miteinander gereinigt werden können.

In einem weiteren Schritt werden die gereinigten Filtertücher in einem Trocknungsschrank thermisch getrocknet. Konkret können dazu wiederum die in dem Korb aufgeschichteten Filtertücher mit dem Korb in den Trocknungsschrank gestellt und dort unter Wärmeeinwirkung und Dampfabzug behandelt werden.

Schließlich erfolgt eine Verpackung der Filtertücher in einer Sterilverpackung. Diese Sterilverpackung weist eine Porendichte auf, die es erlaubt, Keime aus dem Inneren der Sterilverpackung fernzuhalten. So kann etwa eine atmungsfähige Substratseite unter Zwischenlage des Filtertuchs mit einer gegenüberliegenden Deckfolie verbunden, etwa verschweißt werden, was das Endprodukt ergibt.

Auf diese Weise entsteht ein Filterprodukt, welches aus einem gereinigten, sterilen und partikelfreien Filtertuch besteht, welches von einer Sterilverpackung umhüllt ist, die eine spätere Kontaminierung vor dem Einsatz des Filtertuchs verhindert.

In Weiterbildung des Filterprodukts kann der Zuschnitt des Filtermaterials ebenfalls bereits unter Reinraumbedingungen, vorzugsweise der Reinraumklasse ISO 6, stattfinden und unter Absaugung von Schnittgut und abgetrennten Fasern unter Verwendung eines Laserschneidgeräts erfolgen. Dies sorgt für einen exakten Zuschnitt mit sauberen und versiegelten Schnittkanten. Verfärbungen und Verunreinigungen des Filtertuchs durch den Zuschnitt werden damit bereits vermieden.

Im Einzelnen erfordert der Zuschnitt den Schnitt einer Außenkontur, welche zu dem verwendeten Wannensystem passt, sowie den Schnitt von Aussparungen für Haltemittel innerhalb des Wannensystems und zur Aufnahme von Einfüllstutzen. Diese werden beim Zusammensetzen des Wannensystems unter Verwendung des Filtertuchs mithilfe geeigneter Haltemittel dicht verschlossen.

Nach dem Zuschnitt können an dem noch ungereinigten Filtertuch noch grobe Verunreinigungen anhaften, sei es aus dem Vorprozess, sei es vom Zuschnitt selbst. Daher kann mit einigem Vorteil vor der Reinigung im Ultraschallbad ein Absaugvorgang stattfinden, welcher das Filtertuch von groben, aber leicht ablösbaren Verunreinigungen befreit. Hierdurch wird zudem das Ultraschallbad sauber gehalten und das dort verwendete Reinigungsmedium muss seltener ausgetauscht werden.

Hinsichtlich der verwendeten Sterilverpackung besteht eine große Freiheit. Besonders bevorzugt ist aber eine Sterilverpackung, welche aus einer Basisschicht aus einem Polyethylen-Vliesstoff hoher Dichte hergestellt ist, welche unter Zwischenlage des Filtertuchs mit einer transparenten Folie überzogen ist. Dies erlaubt es, in einem zusätzlichen Schritt nach dem Einschweißen des Filtertuchs in die Sterilverpackung, eine Begasung mit einem gasförmigen Sterilisationsmittel vorzunehmen. Während die Struktur des Polyethylen-Vliesstoffs so engporig ist, dass ein Eindringen von Keimen nicht möglich ist, ist es gleichzeitig offenporig genug, um ein Sterilisationsmittel, vorzugsweise Ethylenoxid, durchzulassen. Die Sterilisation kann also in einem solchen Fall auch an dem bereits verpackten Filtertuch vorgenommen werden.

Als Filtertuch eignet sich für die Lyophilisation besonders ein polymeres Gewebe, ein polymerer Filz oder eine polymere Membran. Besondes bevorzugt wird für dieses Anwendungsgebiet als Filtermaterial expandiertes Polytetraflourethylen eingesetzt. Das Herstellungsverfahren ist aber beliebig auf andere Filtertücher für andere Einsatzzwecke übertragbar und für diese auch ausdrücklich mitbeansprucht.

Die vorstehende Erfindung wird im Folgenden anhand eines Ausführungsbeispiels näher erläutert.

Es zeigen
- Figur 1: ein Filterprodukt, welches aus einem in einer Sterilverpackung aufgenommenen Filtertuch besteht, in einer schematischen, perspektivischen Darstellung, sowie
- Figur 2: ein Wannensystem zur Lyophilisation, in welchem ein aus dem Filterprodukt gemäß Figur 1 entnommenes Filtertuch eingesetzt wird, in einer perspektivischen Explosionsdarstellung.

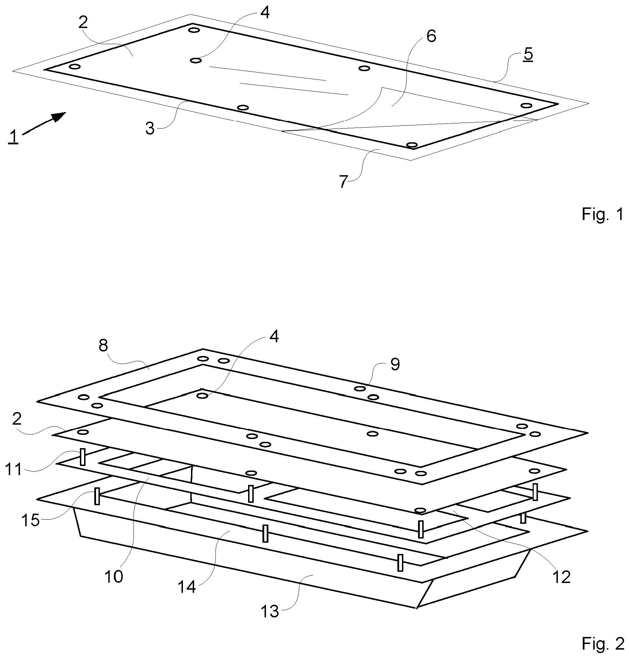

Figur 1 zeigt ein Filterprodukt 1, welches ein Filtertuch 2 umfasst. Dieses Filtertuch 2 ist dadurch hergestellt, dass aus einem Filtermaterial aus expandiertem Polytetraflourethylen (ePTFE) die im Wesentlichen rechteckige Außenkontur 3 des Filtertuchs 2, sowie Aussparungen 4 zum Durchlass von Haltemitteln 11 eines Wannensystems ausgeschnitten werden. Das Ausschneiden erfolgt, bereits unter Reinraumbedingungen, mithilfe eines Laserschneideverfahrens, das an dem polymeren Gewebe für eine Versiegelung der Schnittkanten sorgt und das Verunreinigen mit Fasern vermeidet.

Nach dem Ausschneiden des Filtertuchs 2 wird dieses in einem zweiten Schritt durch Absaugen gereinigt, um grobe Verunreinigungen, die sich leicht lösen lassen, zu entfernen. Dies entlastet den nächsten Schritt, der in einem Ultraschallbad besteht, in welches das Filtertuch 2 eingebracht wird. Hierzu wird eine Mehrzahl von Filtertüchern 2 gemeinsam in einem Korb beabstandet übereinandergeschichtet und der Korb als Ganzes in das Ultraschallbad verbracht. Dort erzeugt die Anregung mit Ultraschall kleine Dampfbläschen in einem das Ultraschallbad füllenden Reinigungsmedium, die unter anderem in der unmittelbaren Nähe des Filtertuchs 2 implodieren. Die dabei frei werdende mechanische Kraft sprengt Verunreinigungen von den Fasern der Filtertücher 2 ab.

Im Nachgang zu dem Ultraschallbad werden die noch nassen Filtertücher mit dem ganzen Korb in einen Trocknungsschrank gebracht und dort getrocknet. Schließlich werden die Filtertücher einzeln in eine Sterilverpackung 5 gelegt, welche aus einem Polyethylen-Vliesstoff 7 und einer darüberliegenden transparenten Folie 6 hergestellt ist. Folie 6 und Vliesstoff 7 werden unter Zwischenlage des Filtertuchs 2 miteinander verbunden, etwa verschweißt, so dass durch die Sterilverpackung 5 hindurch keine Keime mehr von außen an das Filtertuch 2 gelangen können.

Der Polyethylen-Vliesstoff 7 ist jedoch derart gasdurchlässig, dass zwar keine Keime, aber ein Sterilisationsgas hindurchdringen kann. Daher wird das steril verpackte Produkt in einem abschließenden Sterilisationsschritt auch selbst sterilisiert, indem es mit Ethylenoxid begast wird.

Somit ist ein Filterprodukt hergestellt, welches partikelfrei, sowie steril verpackt und sterilisiert vorgehalten werden kann, bis es beispielsweise im Rahmen eines Lyophilisationsverfahrens eingesetzt wird. Dies wird im Folgenden kurz erläutert.

Figur 2 zeigt in einer Explosionsdarstellung ein Wannensystem, das eine elektrisch polierte Wanne 13 aus Edelstahl mit einem breiten Wannenrand 14 umfasst. Diese Wanne 13 nimmt ein zur Gefriertrocknung vorgesehenes Produkt, etwa ein Pharmaprodukt, auf, welches allerdings nicht luftdicht eingeschlossen werden soll. Ein Luftaustausch soll nach oben gewährleistet sein. Die Wanne 13 wird nach oben durch einen Halterahmen abgedeckt, in dem das Filtertuch 2 aufgenommen ist. Hierzu wird zunächst das Filtertuch 2, wie in Figur 1 angedeutet, aus der Sterilverpackung 5 entnommen und auf einen unteren Halterahmen 10 aufgelegt. Aussparungen 4 des Filtertuchs 2 nehmen hierbei Haltemittel 11 des unteren Halterahmens 10 auf, so dass das Filtertuch 2 fest positioniert ist. Ein Stützkreuz 12 im unteren Halterahmen 10 sorgt dafür, dass das Filtertuch 2 nicht durchhängt.

Über dem Filtertuch 2 wird dann ein oberer Halterahmen 8 über die Haltemittel 11 des unteren Halterahmens 10 gelegt, wofür passende Ausnehmungen 9 vorgesehen sind. Mithilfe der Haltemittel, beispielsweise Schraubbolzen, die durch hier nicht gezeigte Flügelmuttern gehalten sein können, wird der obere Halterahmen 8 unter Zwischenlage des Filtertuchs 2 gegen den unteren Halterahmen 10 gedrückt und die Zwischenräume dadurch dicht verschlossen.

Der so gebildete Halterahmen wird schließlich auf den Haltemitteln 15 auf dem Wannenrand 14 aufgesetzt und in gleicher Weise mit Befestigungsmitteln, beispielsweise Flügelmuttern, dicht geschraubt. Das Produkt kann schließlich über eine Einfüllöffnung durch das Filtertuch 2 hindurch in die Wanne eingefüllt werden.

Vorstehend beschrieben ist somit ein Filterprodukt und dessen Herstellungsverfahren, welches mit einem möglichst geringen Aufwand aber gleichzeitig in hoher Qualität steril einsatzfähig ist.

### BEZUGSZEICHENLISTE

- 1: Filterprodukt
- 2: Filtertuch
- 3: Außenkontur
- 4: Aussparung
- 5: Sterilverpackung
- 6: transparente Folie
- 7: Polyethylen-Vliesstoff
- 8: oberer Halterahmen
- 9: Aussparung
- 10: unterer Halterahmen
- 11: Haltemittel
- 12: Stützkreuz
- 13: Wanne
- 14: Wannenrand
- 15: Haltemittel

## Patentansprüche

1. Verfahren zur Herstellung eines partikelfreien, steril verpackten Filterprodukts, welches ein Filtermaterial umfasst, das zu einem Filtertuch (2) zugeschnitten ist,
**dadurch gekennzeichnet, dass** die Herstellung des Filterprodukts (1) die Schritte eines Zuschnitts, einer Vorreinigung durch Absaugen, einer Reinigung des Filtertuchs (2) unter Reinraumbedingungen in einem Ultraschallbad, einer Trocknung des gereinigten Filtertuchs (2), einer Versiegelung des getrockneten Filtertuchs (2) in einer für Keime undurchlässigen Sterilverpackung (5) sowie einer Begasung mit einem gasförmigen Sterilisationsmittel umfasst, wobei die Sterilverpackung (5) des Filterprodukts aus einer Schicht aus einem Polyethylen-Vliesstoff (7) hoher Dichte, welcher derart gasdurchlässig ist, dass zwar keine Keime, aber ein Sterilisationsgas hindurchdringen kann, hergestellt ist, welche unter Zwischenlage des Filtertuchs (2) mit einer transparenten Folie (6) überzogen ist und das steril verpackte Produkt in einem abschließenden Sterilisationsschritt auch selbst sterilisiert wird, indem es mit einem Sterilisationsmittel begast wird.

2. Verfahren gemäß Anspruch 1, **dadurch gekennzeichnet, dass** das Filtermaterial unter Reinraumbedingungen mithilfe eines Lasers zugeschnitten ist.

3. Verfahren gemäß Anspruch 2, **dadurch gekennzeichnet, dass** der Zuschnitt den Schnitt einer Außenkontur (3) sowie den Ausschnitt von Aussparungen (4) für Haltemittel (11) und Einfüllstutzen aus dem Filtermaterial umfasst.

4. Verfahren gemäß einem der Ansprüche 1 bis 3, **dadurch gekennzeichnet, dass** das Sterilisationsmittel Ethylenoxid ist.

5. Verfahren gemäß einem der Ansprüche 1 bis 4, **dadurch gekennzeichnet, dass** das Filtermaterial ein polymeres Gewebe, ein polymerer Filz oder eine polymere Membran, insbesondere expandiertes Polytetraflourethylen ist.

## Claims

1. Method for producing a particle-free, sterile-packaged filter product, which comprises a filter material that is cut to form a filter cloth (2),
**characterized in that** the production of the filter product (1) comprises the steps of cutting, pre-cleaning by suction, cleaning the filter cloth (2) under clean room conditions in an ultrasonic bath, drying the cleaned filter cloth (2), sealing the dried filter cloth (2) in sterile packaging (5) that is impermeable to germs, and gassing with a gaseous sterilizing agent, the sterile packaging (5) of the filter product consisting of a layer of high-density polyethylene nonwoven fabric (7), which fabric is gas-permeable such that no germs can pass through but a sterilization gas can pass through, which layer is covered with a transparent film (6) with the interposition of the filter cloth (2), and the sterile packaged product also being sterilized itself in a final sterilization step by being gassed with a sterilizing agent.

2. Method according to claim 1, **characterized in that** the filter material is cut under clean room conditions using a laser.

3. Method according to claim 2, **characterized in that** the cutting process comprises cutting an outer contour (3) and cutting out recesses (4) for holding means (11) and filler necks from the filter material.

4. Method according to any of claims 1 to 3, **characterized in that** the sterilizing agent is ethylene oxide.

5. Method according to any of claims 1 to 4, **characterized in that** the filter material is a polymer fabric, a polymer felt or a polymer membrane, in particular expanded polytetrafluoroethylene.

## Revendications

1. Procédé de fabrication d'un produit filtrant emballé de manière stérile, sans particules, qui comprend un matériau filtrant découpé pour former une toile filtrante (2),
**caractérisé en ce que** la fabrication du produit filtrant (1) comprend les étapes de découpe, de pré-nettoyage par aspiration, de nettoyage de la toile filtrante (2) dans des conditions de salle blanche dans un bain à ultrasons, de séchage de la toile filtrante (2) nettoyée, de scellage de la toile filtrante (2) séchée dans un emballage stérile (5) imperméable aux germes, et de gazage avec un agent stérilisant gazeux, l'emballage stérile (5) du produit filtrant étant fabriqué à partir d'une couche de non-tissé en polyéthylène haute densité (7) perméable aux gaz de manière à ce qu'aucun germe ne puisse pénétrer, mais qu'un gaz de stérilisation puisse pénétrer, lequel emballage stérile étant recouvert d'un film transparent (6) avec interposition de la toile filtrante (2), et le produit emballé de manière stérile étant également stérilisé lui-même dans une étape de stérilisation finale, par gazage avec un agent stérilisant.

2. Procédé selon la revendication 1, **caractérisé en ce que** le matériau filtrant est découpé dans des conditions de salle blanche à l'aide d'un laser.

3. Procédé selon la revendication 2, **caractérisé en ce que** la découpe comprend la coupe d'un contour extérieur (3) et l'échancrure d'évidements (4) pour des moyens de retenue (11) et des tubulures de remplissage dans le matériau filtrant.

4. Procédé selon l'une des revendications 1 à 3, **caractérisé en ce que** l'agent stérilisant est l'oxyde d'éthylène.

5. Procédé selon l'une des revendications 1 à 4, **caractérisé en ce que** le matériau filtrant est un tissu polymère, un feutre polymère ou une membrane polymère, en particulier du polytétrafluoroéthylène expansé.
